Europäisches Patentamt

European Patent Office

Office européen des brevets

(11) Veröffentlichungsnummer: **0 326 816**

**A1**

## EUROPÄISCHE PATENTANMELDUNG

(21) Anmeldenummer: 89100341.0

(22) Anmeldetag: 10.01.89

(51) Int. Cl.4: **A61K 9/22** , **A61M 31/00**

(30) Priorität: 05.02.88 DE 3803482

(43) Veröffentlichungstag der Anmeldung:
**09.08.89 Patentblatt 89/32**

(84) Benannte Vertragsstaaten:
**AT BE CH DE ES FR GB GR IT LI LU NL SE**

(71) Anmelder: **LTS Lohmann Therapie-Systeme GmbH & Co. KG**
**Irlicherstrasse 55**
**D-5450 Neuwied 12(DE)**

(72) Erfinder: **Müller, Walter Dr.**
**Engerser Strasse 56**
**D-5450 Neuwied 1(DE)**
Erfinder: **Anders, Edzard**
**Herselerstrasse 11**
**D-5303 Bornheim 1(DE)**

(74) Vertreter: **Neidl-Stippler, Cornelia, Dr.**
**Rauchstrasse 2**
**D-8000 München 80(DE)**

(54) Schwimmfähiges orales therapeutisches System.

(57) Die Erfindung betrifft ein schwimmfähiges orales therapeutisches System, bei dem eine Verlängerung der gastrointestinalen Verweilzeit von Medikamenten und eine gesteuerte Abgabe derselben durch Systeme, die spezifisch leichter als die Magenflüssigleit sind, auf dieser schwimmen und schlecht zum tief gelegenen Pförtner gelangen können, erreicht wird, wobei erfindungsgemäß vorgeschlagen wird, mindestens ein Strukturelement (2,7,8) mit Hohlräumen, wie Schäume oder Hohlkörper, einzusetzten.

# Fig. 1

EP 0 326 816 A1

## Schwimmfähiges orales therapeutisches System

Die Erfindung betrifft ein schwimmfähiges orales therapeutisches System.

Orale therapeutische Systeme sind wirkstoffhaltige Einrichtungen, die Wirkstoffe gesteuert an ihre Umgebung abgeben.

Bei oralen therapeutischen Systemen treten neben der Problematik der gesteuerten Wirkstoffabgabe, wie sie von den bekannten transdermalen transmucosalen, sublingualen, nasalen, vaginalen und transplantablen Systemen bekannt sind,zusätzliche Probleme dahingehend auf, das System genügend lange an dem Magen bzw. dem Magen-Darm-Trakt, dem Ort der Wirkstoffabgabe, zu halten. Diese sog. gastrointestinale Verweilzeit ist großen individuellen Schwankungen unterworfen und hängt u.a. von den Ernährungsgewohnheiten des Individuums ab.

Es wurde versucht, Einfluß auf die gastrointestinale Verweilzeit von Medikamenten insbesondere dahingehend, diese zu verlängern, auszuüben. So wurde bspw. vorgeschlagen, Arzneimittelformen einzusetzen, die mit der Magen- bzw. Darmwand verkleben (Drug Development and Industrial Pharmacy, 9(7) 1316-19 (1983)). Es wurde auch versucht, Materialien einzusetzen, die im Magen stark quellen demzufolge den Pförtner nicht passieren können und durch ihr großes Volumen den Magen in einen Sättigungszustand versetzen. Diese unterdrücken die bei nüchterem Magen periodisch auftretenden heftigen peristaltischen Bewegungen, durch die auch größere Nahrungsteilchen in den Darm befördert werden können. Schließlich ist vorgeschlagen worden, Systeme, die spezifisch leichter als die Magenflüssigkeit sind, auf dieser schwimmen und schlecht zum tief gelegenen Pförtner gelangen können, zu diesem Zweck einzusetzen.

So wurde z.B im US-PS 4,167,558 eine schwimmfähige Tablette beschrieben, die allein aufgrund des geringen spezifischen Gewichts ihrer Matrixformulierung in der Magen-Darmflüssigkeit schwimmt; in der US-PS 4,055,178 ist ein mit einer Schwimmkammer versehenes flächenförmiges System vorgeschlagen worden und in den US-PS-en 3,901,232 und 3,786,831 werden, Systeme beschrieben, bei denen sich ein spezifisch leichteres Gewicht erst im Magen durch das Verdampfen einer unterhalb der Körpertemperatur siedenden physiologisch unbedenklichen Flüssigkeit aufbläht.

Die bisher bekannten Lösungen dieses Problems besaßen schwerwiegende Nachteile. Im Falle des US-PS 4,167,558 müssen Matrixmaterialien mit einem hinreichend niedrigen spezifischen Gewicht eingesetzt werden, so daß lediglich eine geringe Auswahl möglich ist. Bei dem mit einer Schwimmkammer versehenen flächenförmigen System der US-PS 4,055,178 sind bestimmte Geometrien vorgegeben, die nicht umgangen werden können. Die in den US-PS-en 3,901,232 und 3,786,831 beschriebenen Systeme sind kompliziert aufgebaut und erfordern einen hohen Fertigungsaufwand.

Es ist demzufolge Aufgabe der Erfindung, neue schwimmfähige oral therapeutische Systeme zu entwickeln, die die Nachteile d.o.g. vermeiden.

Diese Aufgabe wird durch ein gattungsgemäßes System gelöst, das mindestens ein Strukturelement mit Hohlräumen, wie Schäume oder Hohlkörper, aufweist.

Vorteilhafte Weiterbildungen ergeben sich aus den Unteransprüchen.

Es kann vorteilhaft sein, daß die Strukturelemente im System homogen verteilt sind. Das Strukturelement kann auch folienartig sein. Das Strukturelement kann den Wirkstoff umschließen.

Das/die Strukturelement (e) kann ein Polymeres, wie Polyethylen, Polypropylen, Polyamid, Polystyrol, Polyester, Polyacrylat, Polytetrafluorethylen, Polyvinylchlorid, Polyvinylidenchlorid, Copolymer aus den genannten Polymeren zugrundeliegenden Monomeren oder Polysiloxan aufweisen.

Es kann aber auch ein anorganisches Material, beispielsweise Glas oder Keramikmaterial, aufweisen.

Das System weist bei einer bevorzugten Ausführungsform der Erfindung eine Steuermembran für die Wirkstoffabgabe auf.

Es kann eine wirkstoffhaltige Heißschmelzmasse aufweisen, in der die Strukturelemente eingebettet sind.

Das/die Strukturelement(e) kann in einem wirkstoffhaltigen Formkörper eingebettet sein.

Der Formkörper kann ein Hydrogel sein bzw. ein Hydrogel bei Kontakt mit der Magen- Darmflüssigkeit bilden.

Das System kann schwimmfähige Untersysteme aufweisen, deren zentraler Teil Strukturelemente mit hohem Hohlraumanteil sind, wobei diese Strukturelemente mit einem wirkstoffhaltigen Überzug versehen sind oder Wirkstoffe aufweisen.

Die Untersysteme können in einer unter physiologischen Bedingungen löslichen Kapsel eingeschlossen sein.

Die schwimmfähigen Untersysteme können auch durch ein Bindemittel zu einem Formkörper vereinigt sein, wobei ggf. die Untersysteme bei Kontakt mit der Magen- und/oder Darm flüssigkeit unter Auflösung des Bindemittels freigegeben werden können.

Das System kann mehrschichtig sein, wobei mindestens eine Schicht ein schwimmfähiges Strukturelement ist.

Das System kann vor Applikation zusammengefaltet oder gerollt sein und unter Magenbedingungen entfalt- bzw. entrollbar.

Vorteilhafterweise ist das System vollständig oder teilweise unter physiologischen Bedingungen abbaubar.

Überraschenderweise wurde gefunden, daß durch den Einsatz von Materialien, die einen hohen Hohlraumanteil bzw. Gaseinschlüsse besitzen, orale Systeme mit einem geringen spezifischen Gewicht hergestellt werden können. Als Materialien eignen sich bspw. Schäume oder Hohlkugeln, die aus unterschiedlichsten Materalien hergestellt sein können, bspw. aus allen thermoplastischen Polymeren, natürlichen Polymeren oder auch anorganischen Verbindungen, wie Gläsern und keramischen Materialien.

Im Handel sind insbesondere schaumartige oder auch als mikroporös bezeichnete Strukturen aus thermoplastischen Polymeren in Form von Pulvern, Folien, Stäben und Schläuchen kommerziell erhältlich.

Das Eindringen von Wasser in Poren kann durch eine ausreichend geringe Porengröße über Kapilareffekte vermieden werden oder auch durch die Verwendung hydrophober Polymere, die den Zutritt von Wasser, insbesondere in Hohlräumen·im inneren der Polymeren verhindern; vorausgesetzt die Porengröße ist ausreichend gering.

Es können auch Glaskugeln mit geringen Durchmessern, wie sie im Handel erhältlich sind, als Hohlkörper zur Erleich terung des spezifischen Gewichts des Systems eingesetzt werden.

In der DE-OS 32 15 211 ist ein Verfahren zur Herstellung von mit Wirkstoff beladenen mikroporösen Pulvern beschrieben worden. Hierbei wird bei erhöhter Temperatur eine homogene Polymerlösung in ein Gas zerstäubt. Dabei entmischen sich Polymer und Lösemittel;nach Entfernen des Lösemittels bleiben Teilchen mit mikroporöser Struktur zurück. Falls während der Herstellung der mikroporösen Teilchen Wirkstoff zugesetzt wird oder der Porenbildner selbst derWirkstoff ist, erhält man wirkstoffbeladene mikroporöse Pulver.

In der EP-A2-0 146 740 ist ein Verfahren zur Herstellung von Formkörpern mit mikroporöser Struktur aus mikroporösen Pulvern über ein Preßvorgang beschrieben. Es werden Möglichkeiten angegeben, die mikroporösen Formkörper vor dem Verpressen mit Wirkstoffen zu beladen.

In der EP-A2-0 162 492 wird eine Tablette mit einer die Wirkstoffabgabe steuernden Membran beschrieben, die aus mikroporösen Pulvern durch einen Preßvorgang hergestellt ist.

Demgegenüber zeichnet sich die Erfindung dadurch aus, daß hier mikorpöröse Stoffe bzw. Strukturen eingesetzt werden, die geringes spezifisches Gewicht besitzen, und inbesondere unter Magenbedingungen über ein hinreichend großen Zeitraum aufrecht erhalten. In keinem Fall werden alle Hohlräume der Strukturelemente mit Wirkstoffen beladen; es bleibt stets hinreichend Hohlraum frei, um ein niedriges spezifisches Gewicht zur Aufrechterhaltung der Schwimmfähigkeit des Systems zu erhalten.

Ein besonderer Vorteil der Erfindung besteht darin, daß Systeme mit bis zu 70 Vol.-%und bis zu 80 Gew.-% mit Wirk stoff beladen werden können. Es ist auch möglich, die mit Wirkstoff beladenen Systeme zusätzlich mit einer Steuerschicht, bspw. einer über die Porengröße steuernden Membran oder aber auch einer über die Diffusionsgeschwindigkeit steuernden Membran zu überziehen, um weitere Steuerfunktionen ausüben zu können.

Nachfolgend wird die Erfindung anhand der begleitenden Zeichnung erläutert, in der zeigt:

Fig. 1 ein orales therapeutisches System, wobei Hohlraumstrukturelemente 2 in einer wirkstoffhaltigen Matrix 1 verteilt sind;

Fig. 2 eine erfindungsgemäße Mehrschichttablette;

Fig. 3 ein tablettenförmiges System mit einem mikroporösen Strukturelement als Tablettenkern;

Fig. 4a ein mehrere Untersysteme aufweisendes System;

Fig. 4b einen Schnitt durch eine bevorzugte Ausführungsform eines Untersystems der Fig. 4a;

Fig. 4c einen Schnitt durch eine weitere bevorzugte Ausführungsform eines Untersystems gemäß Fig. 4a;

Fig. 5a ein flächenförmiges System;

Fig. 5b ein Mehrschichtsystem, bei dem eine Schicht aus dem Strukturelement besteht;

Fig. 6a ein Zwischenprodukt zur Herstellung des in der Fig. 6b dargestellten Systems; und

Fig. 6b ein weiteres bevorzugtes therapeutisches orales System, wobei das schwimmfähige Strukturelement ringförmig eine wirkstoffhaltige Zubereitung 1 umschließt.

Fig. 7 die Freisetzungskinetik eines erfindungsgemäßen Systems gemäß Beispiel 1 in einer Auftragung freigesetzter Wirkstoff (mg) gegen die Zeit (h)

Fig. 8 die Freisetzungskinetik eines erfindungsgemäßen Systems gemäß Beispiel 2 in einer Auftragung freigesetzter Wirkstoff (mg) gegen die Zeit (h)

Fig. 9 die Freisetzungskinetik eines erfindungsgemäßen Systems gemäß Beispiel 3 in einer Auftragung freigesetzter Wirkstoff (mg) gegen die Zeit (h)

Fig. 10 die Freisetzungskinetik eines erfindungsgemäßen Systems gemäß Beispiel 4 in einer Auftragung freigesetzter Wirkstoff (mg) gegen die Zeit (h)

Fig. 11 die Freisetzungskinetik eines erfindungsgemäßen Systems gemäß Beispiel 5 in einer Auftragung freigesetzter Wirkstoff (mg) gegen die Zeit (h)

Fig. 12 die Freisetzungskinetik eines erfindungsgemäßen Systems gemäß Beispiel 6 in einer Auftragung freigesetzter Wirkstoff (mg) gegen die Zeit (h)

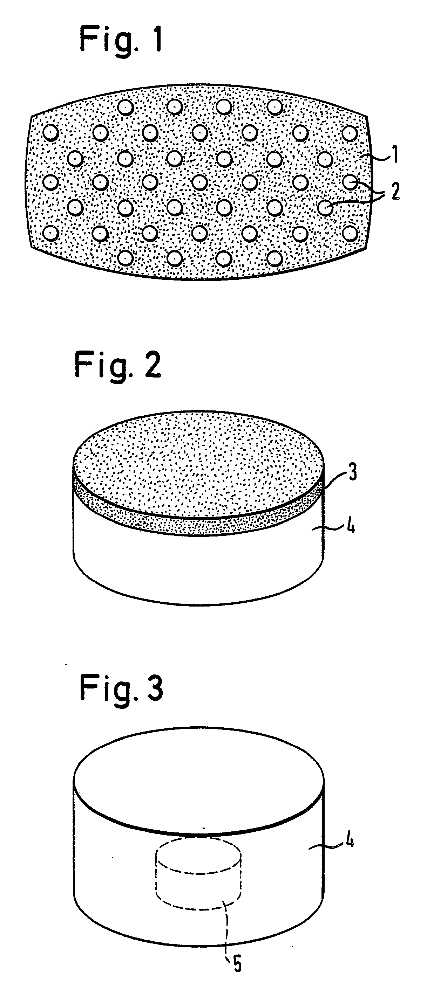

In Fig. 1 ist eine bevorzugte Ausführungsform der Erfindung in Form einer sog. Gerüsttablette dargestellt. Eine derartige Gerüsttablette besteht aus einer unter den im Magen herrschenden physiologischen Bedingungen nicht oder nur langsam desintegrierbaren Matrixformulierung. Sie kann bspw. dadurch hergestellt werden, daß Granulate mit permeablen Acrylharzen beschichtet und durchsetzt werden, und die Partikel sodann ohne zusätzliche Füllstoffe verpreßt werden. Anstelle von Acrylharzen können auch andere, meist hochmolekulare Hilfstoffe verwendet werden, die in den Verdauungssäften nur begrenzte Löslichkeit besitzen. Mischt man dem Granulat vor Verpressung eine ausreichende Menge an Hohlkörpern oder schaumartigen Strukturelementteilchen (2) bei, wird das spezifische Gewicht der Tablette soweit erniedrigt, daß sie in der Magenflüssigkeit schwimmt. Es ist auch möglich, eine derartige Tablette herzustellen, in dem ein kristalliner Wirkstoff in Mischung mit mikroporösen Strukturelementpulvern (2) verpresst wird. Es ist auch möglich, Wirkstoffpartikel vor dem Verpressen separat mit Steuermembranen, bspw. Diffusionsmembranen, durch ansich bekannte Verfahren, wie Besprühen od. dgl., zu versehen. Selbstverständlich können erfindungsgemäße Tabletten, auch übliche Hilfstoffe, wie wasserunlösliche oder quellbare Substanzen, bspw. Cellulosederivate, Polymere, Fette, Wachse oder physiologisch unbedenkliche Heiß-Schmelzmassen aufweisen. Eine solche Tablette kann, inbesondere wenn sie aus einer Heiß-Schmelzmasse hergestellt ist, bei Raumtemperatur oder leicht erhöhten Temperaturen eine ungenügende mechanische Festigkeit besitzen und in diesem Falle mit einen im Magen schnell löslichen Überzug versehen werden. Aus derartigen Systemen entweicht der Wirkstoff hauptsächlich über passive Diffusion. Ein derartiges System kann nun durch Zugabe von Strukturelementenmaterialien mit hohem Hohlraumanteil geeigneter Größe, Art und Menge sicher schwimmfähig gemacht werden.

In Fig. 2 ist ein tablettenförmiges therapeutisches System, dargestellt, das zwei Schichten (3,4) aufweist, wobei die eine Schicht 3 das mikroporöse Strukturelement ist, das die Aufgabe hat, das Gesamtsystem schwimmfähig zu machen. Diese Tablette kann bspw. in vorteilhafter Weise in einem einzigen Preßvorgang hergestellt werden, denkbar ist aber auch, die beiden Tablettenteile getrennt herzustellen und diese anschließend zusammenzufügen. Es ist dabei möglich, als Schwimmhilfe einfach einen Stanzling aus mikroporöser Folie einzusetzen, der bspw. mit dem wirkstoffhaltigen Systemteil 4 durch Verkleben verbunden werden kann.

In Fig. 3 ist ein weiteres tablettenförmiges orales therapeutisches System schematisch gezeigt, bei dem ein mikroporöser Kern 5 als Schwimmhilfe für die wirkstoffhaltige Matrix 4 vorgesehen ist und von dieser allseitig umschlossen ist.

Auch diese Tablette kann in vorteilhafter Weise in einem einzigen Tablettiervorgang hergestellt werden.

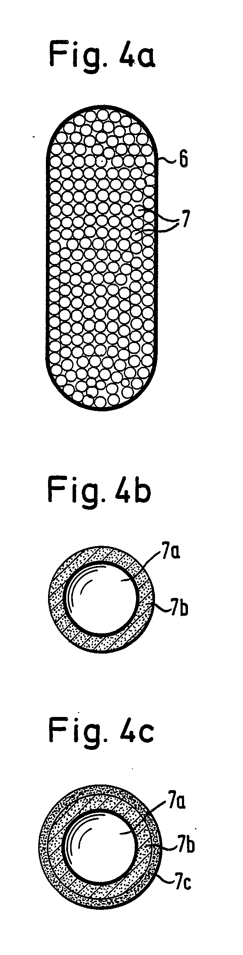

Fig. 4 ist eine weitere bevorzugte Ausführungsform erfindungsgemäßer schwimmfähiger therapeutischer Systeme dargestellt, wobei eine Vielzahl von schwimmfähigen Untersystemen 6 in einer im Magen löslichen Kapsel enthalten sind. Bevorzugte Untersysteme 7 des Gesamtsystems der Fig. 4a sind als Schnitt in Fig. 4b dargestellt. Dabei ist der mit dem Bezugszeichen 7a bezeichnete Kern ein etwa kugelförmiger Hohlkörper oder kugelförmiges Schaumteilchen, das mit einem wirkstoffhaltigen Überzug 7b versehen ist. Die Wirkstofffreisetzung läßt sich bei dieser Ausführungsform eines Untersystems 7a über die Zusammensetzung der Wirkstoffzubereitung, der Dicke des wirkstoffhaltigen Überzugs, die Gesamtoberfläche und die Wirkstoffkonzentration steuern. Wie in Fig. 4c dargestellt, kann bei einer weiter bevorzugten Ausführungsform zusätzlich zu den in Fig. 4b dargestellten Schichten noch eine Steuermembran 7c aufgebracht sein.

Anstelle der Verwendung einer Kapsel als Behältnis für die Untersysteme 7 ist es auch möglich, die Untersysteme selbst durch einen im Magen löslichen bzw. auflösbaren Binder, der den Zusammenhalt des Gesamtsystems bis zur Applikation gewährleistet, zu verbinden.

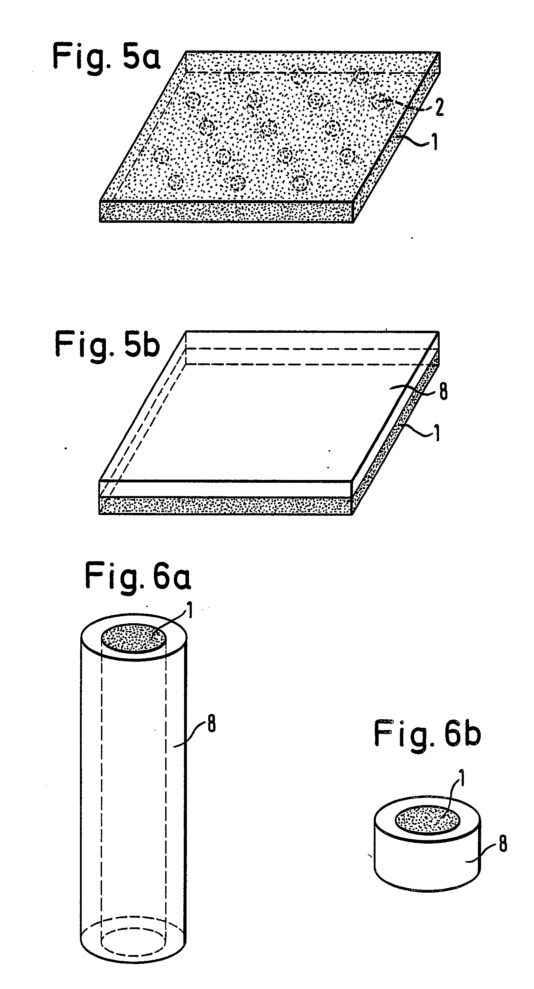

In Fig. 5a ist ein flaches, erfindungsgemäßes System dargestellt, das beispielsweise aus einem physiologischen unbedenklichen, Hohlräume aufweisenden Polymermaterial 2 und Hilfsstoffen 1 besteht. Vor Applikation ist es bspw. zusammengerollt oder zusammengefaltet und kann auch in einer Kapsel verpackt sein. Der Wirkstoff wird sodann bei Applikation entweder durch Diffusion freigesetzt oder dadurch, daß das Polymer unter physiologischen Bedingungen abbaubar ist. Durch die eingearbeiteten Hohlräume ist das System schwimmfähig.

In Fig. 5b ist ein zweischichtiges Laminat dargestellt, bei dem die Schicht 8 Hohlräume aufweist und als Schwimmhilfe fungiert. Sie ist hier bevorzugt eine Folie, die durch ihre schaumartige Struktur über einen hohen Hohlraumanteil verfügt, während die Schicht 1 eine wirkstoffhaltige Matrix ist. Selbstverständlich ist es möglich, noch weitere Schichten unterschiedlicher Zusammensetzung vorzusehen, ohne vom Erfindungsgedanken abzuweichen.

In Fig. 6a ist eine röhrenförmige Struktur mit einer hohlraumhaltigen Folie 8 als Röhrenmaterial dargestellt, die ein wirkstoffhaltiges Matrixmaterial 1, das in der Röhre vorgesehen ist, ringförmig umschließt.

Das wirkstoffhaltige Material 1 kann bspw. in einen Heiß-Schmelzmasse oder ähnlichen eingebracht werden und das System anschließend in die in Fig. 6b dargestellten Scheiben geschnitten werden. Die in Fig. 6b dargestellten Systeme können selbstverständlich in an sich bekannter Weise zusätzlich steuernde Membranen oder andere, im Magen lösliche Mantelmaterialien aufweisen, bzw. von diesen umschlossen sein.

Erfindungsgemäß ist es also u.a. möglich, Strukturelemente mit hohem Hohlraumanteil homogen in einem Gesamtsystem verteilt: als zentraler Teil von vielen kleinen Untersy stemen; in Form einer Folie als Teil eines schichtenaufweisenden Körpers; als zentralen Teil einer Tablette; als Teil einer Mehrschichttablette oder aber als dem Hüllung vorzusehen, um dadurch ein orales therapeutisches System schwimmfähig zu machen.

Einfache orale therapeutische Systeme, bei denen bspw. die hohlraumaufweisenden Strukturelemente homogen im Gesamtsystem verteilt vorliegen, können durch an sich bekannte Extrusionsverfahren, Spritzgußverfahren oder Gießverfahren hergestellt werden.

Es ist auch möglich, derartige Systeme über Preßvorgänge herzustellen.

Es ist ein getrennte Herstellung des wirkstoffhaltigen Teils der Tablette und der Schwimmhilfe möglich, die als bspw. durch Verkleben und Heißsiegeln zu einem Gesamtsystem vereinigt werden.

Die Schwimmhilfe selbst kann, wie auch andere Teile des Systems, über Preß-/Stanz- oder Extrusionsvorgänge hergestellt werden.

Nachfolgend sollen bevorzugte Ausführungsformen der Erfindung anhand von Beispielen erläutert werden.

Beispiel 1

417 g Theophyllin, mit 78 mg eines Ethylen-Vinylacetat-Copolymeren (unter der Bezeichnung EVATANE 28.800 von ICI erhältlich) gecoatet, werden mit 171 mg Polyamid-12-Schaum ACCUREL EP 900) homogenisiert und auf ein konstantes Volumen von 0,74 ccm verpreßt. Die Dichte der Preßkörper betrug 0.8 g/ccm.

Jeder Preßkörper enthielt etwa 420 mg Theophyllin.

Die Theophyllin-Freisetzung aus dem Preßkörper wurde in 600 ml künstlichem Magensaft bei 37 Grad Celsius mit der Methode USP "Rotating Basket" untersucht. Es zeigte sich, daß die Freisetzung in etwa nach 24 h vollständig erfolgt war, wobei nach einer relativ schnellen Abgabe von etwa 270 mg Theophyllin, also etwa 64 % des Theophyllins in den ersten 8 Stunden, nur noch eine langsame weitere Abgabe erfolgte.

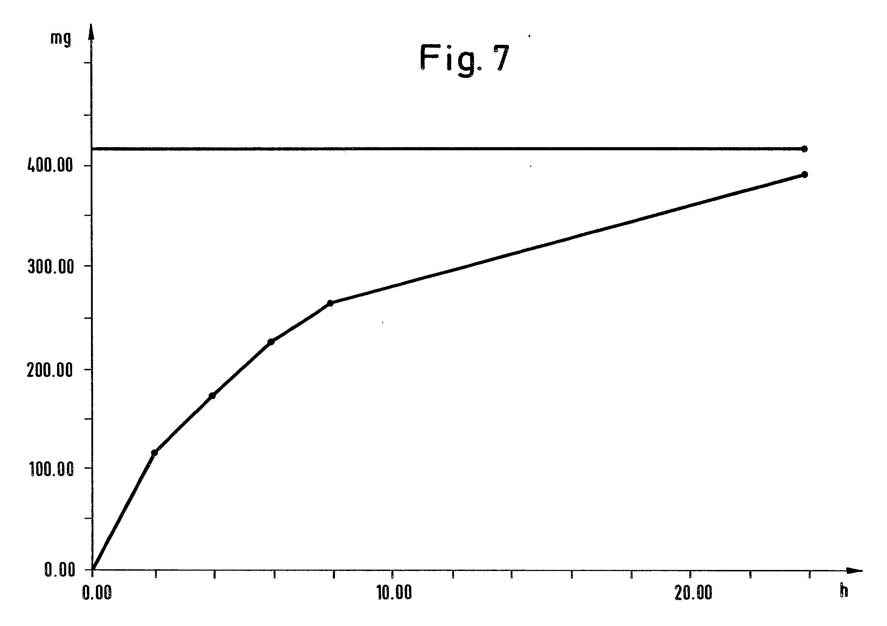

Das Ergebnis des Freisetzungsversuches ist in Fig.7 dargestellt.

Beispiel 2

409 mg Theophyllin, mit 11 mg eines Acrylharzes (Eudragit RL 100 der Fa Röhm Pharma) gecoatet, werden in ein Preßwerkzeug gefüllt, glattgedrückt und gemeinsam mit den danach eingefüllten 180 mg Polypropylenschaumpulver (ACCUREL EP 100, < 200 μm) auf ein konstantes Volumen von 0.74 ccm verpreßt. Der Preßling wies eine Dichte von 0.8 g/ccm und einen Gehalt von 409 mg Theophyllin auf.

Die Theophyllin-Freisetzung aus dem Preßkörper wurde in 600 ml künstlichem Magensaft bei 37 Grad Celsius mit der Methode USP "Rotating Basket" untersucht. Es zeigte sich, daß die Freisetzung in etwa nach 24 h vollständig erfolgt war, wobei nach einer mit relativ konstanter Geschwindigkeit erfolgten schnellen Abgabe von ca 300 mg Theophyllin, also ca 70 % des Theophyllins in den ersten 8 Stunden nur noch eine langsame weitere Abgabe erfolgte.

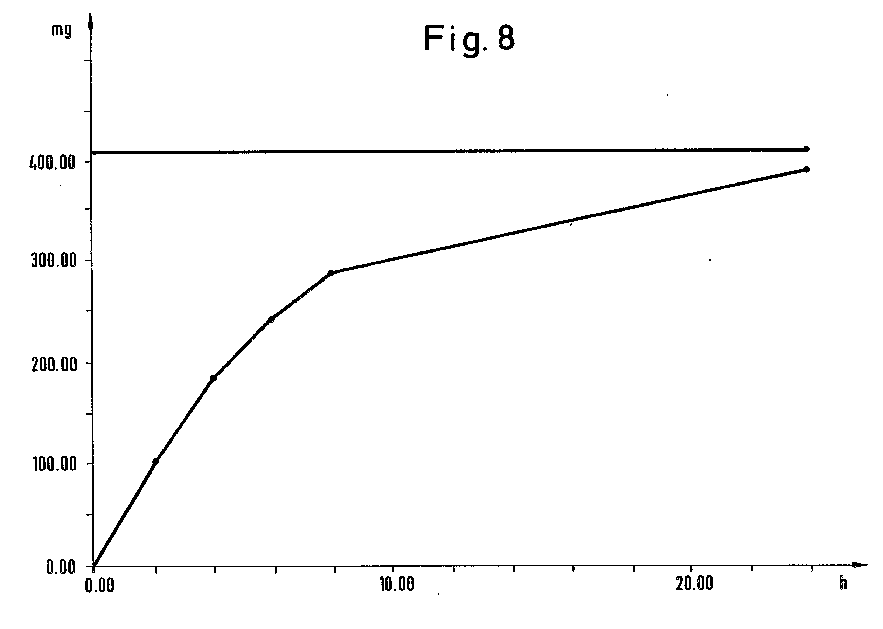

Das Ergebnis der Messung ist in Fig. 8 dargestellt.

Beispiel 3

409 mg Theophyllin, mit 11 mg eines Acrylharzes (Eudragit RL 100 der Fa Röhm Pharma) gecoatet, werden in ein Preßwerkzeug gefüllt, glattgedrückt und gemeinsam mit einer entsprechend gestanzten Polypropylenschaumfolie ( Fa. ACCUREL) auf ein konstantes Volumen von 0.74 ccm verpreßt. Der Preßling wies eine Dichte von 0.,8 g/ccm auf und einen Theophyllin-Gehalt von etwa 409 mg Theophyllin.

Die Theophyllin-Freisetzung aus dem Preßkörper wurde in 600 ml künstlichem Magensaft bei 37 Grad Celsius auf Freisetzung mit der Methode USP "Rotating Basket" untersucht. Es zeigte sich, daß nach einer mit relativ konstanter Geschwindigkeit erfolgten schnellen Abgabe von insgesamt etwa 250 mg Theophyllin, also etwa 60 % des Gesamttheophyllins in einem Preßkörper, in den ersten 8 Stunden, danach nur noch eine langsame weitere Abgabe erfolgte.

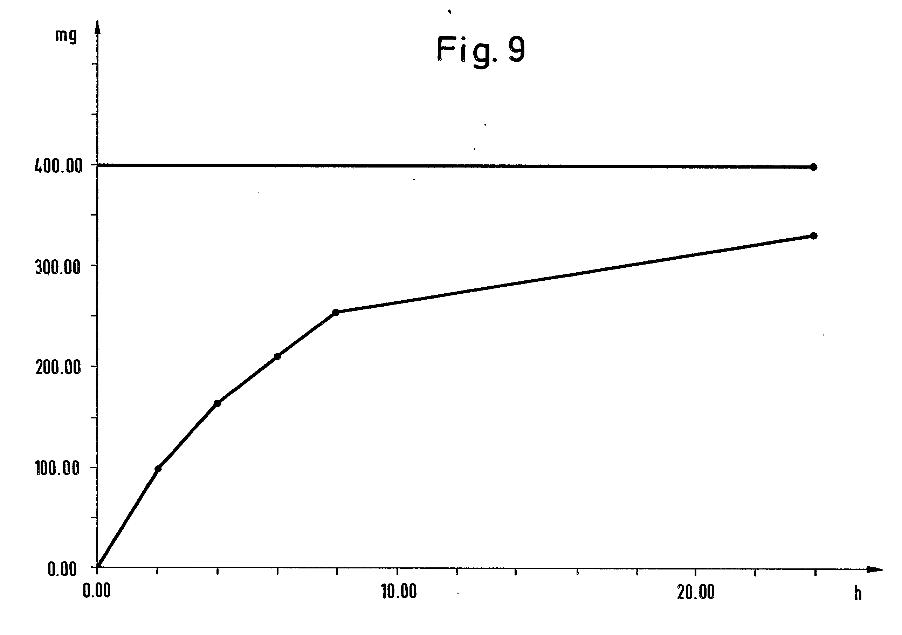

Das Ergebnis der Messung ist in Fig. 9 verdeutlicht.

Beispiel 4

Durch Abbaugranulierung (Naßgranulierung) werden 900 mg eines Schüttelgranulats mit der Korngröße 15 Mesh/ASTM folgender Zusammensetzung hergestellt:

430 mg Theophyllin; 172 mg Polypropylenschaumpulver (ACCUREL EP 100, <200 µm), 298 mg eines Acrylharzes (Eudragit RS 100, erhältlich von der Fa Röhm Pharma). Dieses Granulat wurde in eine handelsübliche Gelatine-Steckkapsel Nr. 00 (CAPSUGEL) eingefüllt, sodaß sich in jeder Kapsel etwa 430 mg Theophyllin befanden.

Zur Untersuchung der Freisetzung von Theophyllin aus dieser Darreichungsform wurde die mit Granulat gefüllte Steckkapsel in 600 ml künstlichem Magensaft bei 37 Grad Celsius mit der Methode USP "Rotating Basket" untersucht. Es zeigte sich, daß nach einer mit relativ konstanter Geschwindigkeit innerhalb etwa 8 Stunden erfolgten schnellen Abgabe von insgesamt etwa 350 mg Theophyllin, also von etwa 81 % des Gesamt-Theophyllins in dieser Zubereitung, nur noch eine langsame weitere Abgabe erfolgte.

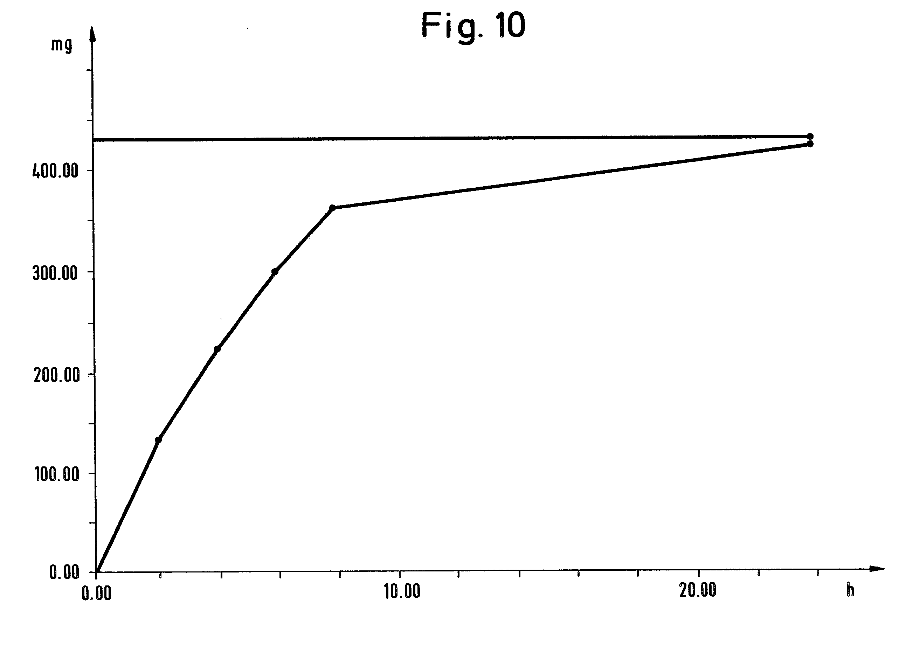

Die Ergebnisse dieses Versuchs sind in Fig. 10 dargestellt.

Beispiel 5

Zu einer bei 100 Grad Celsius geschmolzenen, homogenisierten Mischung aus 7 g Bienenwachs, 10,5 g Carnaubawachs, 17,5 g Polyisobuten (Oppanol B 15/1 der BASF), 10 g eines nichtionischen Tensids auf Basis von Polyethylenglykolethern von langkettigen Alkoholen (Brij 700 der Fa Atlas Chemie) und 2 g Polyethylenglykol (PEG 400) werden unter intensivem Rühren erst 3 g Tylopur MHB 3000 P und 35 g Theophyllin, dann 2,5 g Glashohlraumkugeln (Q-Cel 500) gegeben. Die Masse wird in eine Teflonform gegossen und abgekühlt. Durch Ausstanzen werden die einzelnen oralen therapeutischen Systeme mit jeweils etwa 150 mg Theophyllin erhalten.

Diese oralen Systeme wurden in 600 ml künstlichem Magensaft bei 37 Grad Celsius auf Freisetzung von Theophyllin mit der Methode USP "Rotating Basket" untersucht. Es zeigte sich, daß nach einer mit relativ konstanter Geschwindigkeit erfolgten schnellen Abgabe von insgesamt etwa 125 mg Theophyllin in den ersten 8 Stunden, also von 83 % des Gesamt-Theophyllins in dieser Zubereitung, praktisch keine weitere nennenswerte Freisetzung erfolgte.

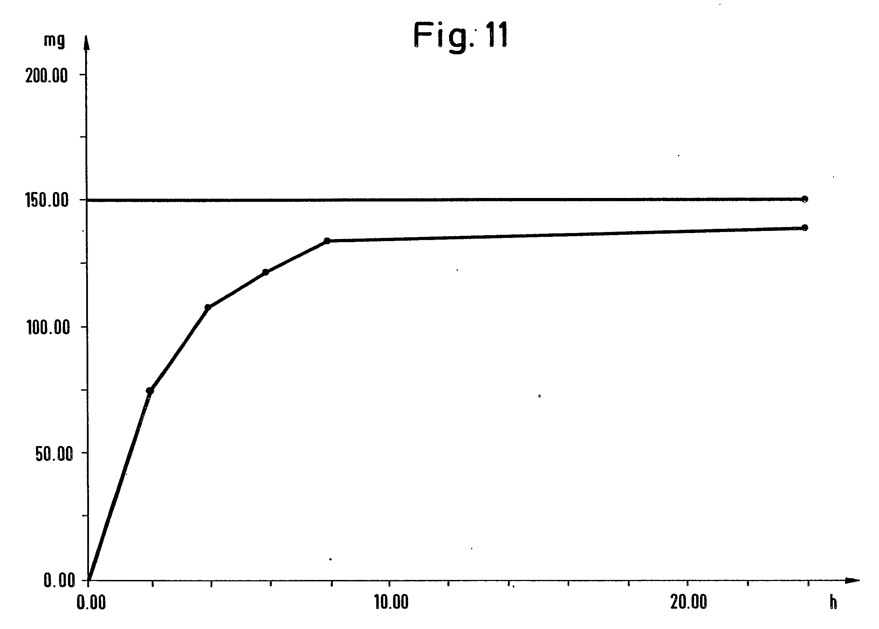

Die Ergebnisse dieser Untersuchung sind in Fig.11 dargestellt.

Beispiel 6

100 g einer Heißschmelzmasse aus 28,5 g Bienenwachs, 28,5 g Staybelite Ester 10E, 20.0 g Theophyllin, 10.0 g Poly ethylenglykol (PEG 1000, 10.0 g Tylopur MH 4000 P und 3,0 g eines nichtionogenen Tensids auf Basis von Polyethylenglykolethern von langkettigen Alkoholen (Brij 76 der ATLAS CHEMIE) wurden bei 80 Grad Celsius unter Vakuum in einen Polypropylenschlauch (D/i: 5,5 mm D/a: 8,5 mm; hergestellt von der Fa ACCUREL, gesogen. Nach Abkühlen erhält man die einzelnen oralen therapeutischen Systeme durch Schneiden.

Die Dichte der hergestellten Systeme betrug 0.65 g/ccm, und sie hatten einen Theophyllingehalt von etwa ca 52 mg Theophyllin pro Einheit.

Die Theophyllin-Freisetzung aus diesen oralen Systemen wurde in 600 ml künstlichem Magensaft bei 37 Grad Celsius nach der Methode USP "Rotating Basket" untersucht. Es zeigte sich, daß nach einer mit relativ konstanter Geschwindigkeit erfolgten schnellen Abgabe über etwa 8 h von etwa 30 mg Theophyllin, also von 57 % des Gesamt-Theophyllins in dieser Zubereitung praktisch keine weitere nennenswerte Freisetzung erfolgte.

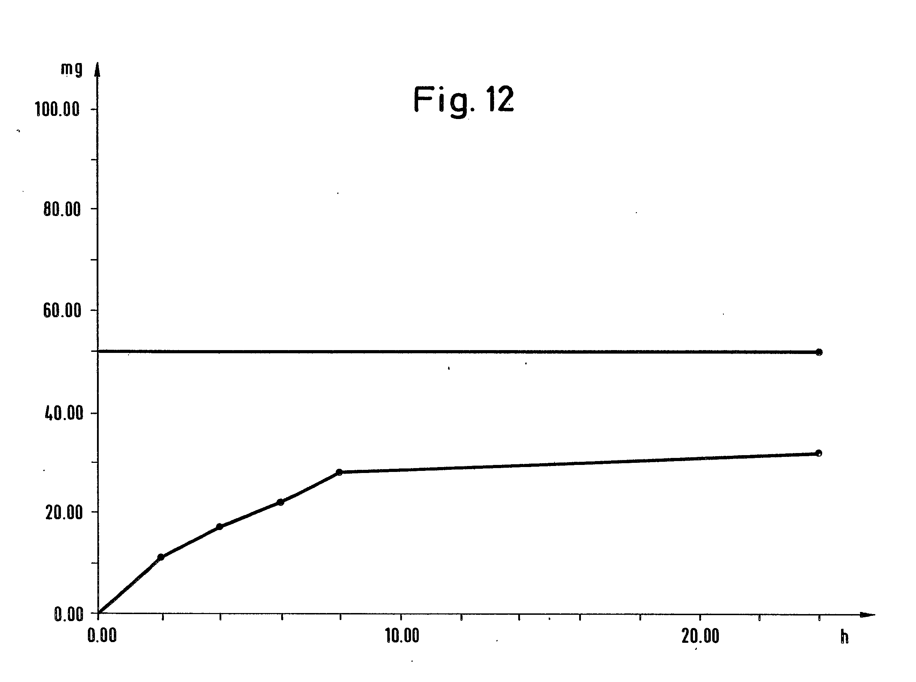

Die Ergebnisse dieses Versuchs sind in Fig. 12 dargestellt.

**Ansprüche**

1. Schwimmfähiges orales therapeutisches System, gekennzeichnet durch mindestens ein Strukturelement (2,7,8) mit Hohlräumen, wie Schäume oder Hohlkörper.

2. System nach Anspruch 1, dadurch gekennzeichnet, daß die Strukturelemente (12) im System homogen verteilt sind.

3. System nach Anspruch 1, dadurch gekennzeichnet, daß das Strukturelement folienartig ist.

4. System nach Anspruch 1, dadurch gekennzeichnet, daß das Strukturelement den Wirkstoff umschließt.

5. System nach einem der vorangehenden Ansprüche, dadurch gekennzeichnet, daß das/die Strukturelement (e) ein Polymeres, wie Polyethylen, Polypropylen, Polyamid, Polystyrol, Polyester, Polyacrylat, Polytetrafluorethylen, Polyvinylchlorid, Polyvinylidenchlorid, Copolymer aus den genannten Polymeren zugrundeliegenden Monomeren oder Polysiloxan aufweist.

6. System nach einem der Ansprüche 1 bis 4, dadurch gekennzeichnet, daß das/die Strukturelement(e) ein anorganisches Material, bspw. Glas oder Keramikmaterial, aufweist.

7. System nach einem der vorangehenden Ansprüche, dadurch gekennzeichnet, daß es eine Steuermembran (7c) für die Wirkstoffabgabe aufweist.

8. System nach einem der vorangehenden Ansprüche, dadurch gekennzeichnet, daß es eine wirkstoffhaltige Heißschmelzmasse (1) aufweist, in der die Strukturelemente eingebettet sind.

9. System nach einem der Ansprüche 1 bis 7, dadurch gekennzeichnet, daß das/die Strukturelement(e) in einem wirkstoffhaltigen Formkörper eingebettet ist/sind.

10. System nach Anspruch 9, dadurch gekennzeichnet, daß der Formkörper ein Hydrogel ist bzw. ein Hydrogel bei Kontakt mit der Magen/Darmflüssigkeit bildet.

11. System nach Anspruch 1, dadurch gekennzeichnet, daß es schwimmfähige Untersysteme aufweist, deren zentraler Teil Strukturelemente mit hohem Hohlraumanteil sind, wobei diese Strukturelemente mit einem wirkstoffhaltigen Überzug versehen sind oder Wirkstoffe aufweisen.

12. System nach Anspruch 11, dadurch gekennzeichnet, daß die Untersysteme in einer unter physiologischen Bedingungen löslichen Kapsel eingeschlossen sind.

13. System nach Anspruch 11 oder 12, dadurch gekennzeichnet, daß die schwimmfähigen Untersysteme durch ein Bindemittel zu einem Formkörper vereinigt sind, wobei ggf. die Untersysteme bei Kontakt mit der Magen- und/oder Darmflüssigkeit unter Auflösung des Bindemittels freigegeben werden können.

14. System nach einem der vorangehenden Ansprüche, dadurch gekennzeichnet, daß es mehrschichtig ist und mindestens eine Schicht ein schwimmfähiges Strukturelement ist.

15. System nach einem der Ansprüche 3, 4 oder 14, dadurch gekennzeichnet, daß es vor Applikation zusammengefaltet oder gerollt ist und unter Magenbedingungen entfalt- bzw. entrollbar ist.

16. System nach einem der vorangehenden Ansprüche, dadurch gekennzeichnet, daß es vollständig oder teilweise unter physiologischen Bedingungen abbaubar ist.

Neu eingereicht / Newly filed
Nouvellement déposé

# Fig. 1

# Fig. 2

# Fig. 3

## Fig. 4a

6

7

## Fig. 4b

7a

7b

## Fig. 4c

7a

7b

7c

Neu eingereicht / Newly file
Nouvellement déposé

Fig. 5a

2

1

Fig. 5b

8

1

Fig. 6a

1

8

Fig. 6b

1

8

Fig. 7

Neu eingereicht / Newly filed
Nouvellement déposé

EP 0 326 816 A1

Fig. 8

Neu eingereicht / Newly filed
Nouvellement déposé

EP 0 326 816 A1

Fig. 9

Neu eingereicht / Newly filed
Nouvellement déposé

EP 0 326 816 A1

Neu eingereicht / Newly filed
Nouvellement déposé

Fig. 10

mg

400.00

300.00

200.00

100.00

0.00

0.00

10.00

20.00

h

Neu eingereicht / Newly fil
Nouvellement déposé

Fig. 11

Fig. 12

Neu eingereicht / Newly filed
Nouvellement déposé

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 263 744 (EISAI CO., LTD)<br>* Insgesamt *<br>--- | 1,5,7,9,16 | A 61 K 9/22<br>A 61 M 31/00 |
| Y | EP-A-0 090 560 (MINNESOTA MINING AND MANUFACTURING CO.)<br>* Ansprüche 1-10 *<br>--- | 1-16 | |
| P,Y | CHEMICAL ABSTRACTS, Band 109, 31. Oktober 1988, Seite 446, Nr. 156279c, Columbus, Ohio, US; JP-A-63 22 014 (SHIONOGI & CO., LTD) 29-01-1988<br>* Zusammenfassung *<br>--- | 1-16 | |
| D,Y | EP-A-0 162 492 (THIEMANN ARZNEIMITTEL GmbH)<br>* Ansprüche 1-9 *<br>----- | 1-16 | |

**RECHERCHIERTE SACHGEBIETE (Int. Cl.4)**

A 61 K
A 61 M

Der vorliegende Recherchenbericht wurde für alle Patentansprüche erstellt

| Recherchenort | Abschlußdatum der Recherche | Prüfer |
|---|---|---|
| DEN HAAG | 10-03-1989 | TZSCHOPPE, D.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)